(19) Europäisches Patentamt

European Patent Office

Office européen des brevets

(11) **EP 1 285 661 B1**

(12) **EUROPEAN PATENT SPECIFICATION**

(45) Date of publication and mention
of the grant of the patent:
**14.06.2006 Bulletin 2006/24**

(21) Application number: **01919903.3**

(22) Date of filing: **12.04.2001**

(51) Int Cl.:
*A61K 36/02* [(2006.01)]    *A61K 8/97* [(2006.01)]
*A61Q 5/02* [(2006.01)]    *A61Q 19/00* [(2006.01)]
*A61Q 19/08* [(2006.01)]    *C09K 15/34* [(2006.01)]
*A61P 29/00* [(2006.01)]    *A61P 17/18* [(2006.01)]
*A61P 39/06* [(2006.01)]

(86) International application number:
**PCT/JP2001/003148**

(87) International publication number:
**WO 2001/089547 (29.11.2001 Gazette 2001/48)**

(54) **USE OF AN ANTIOXIDANT COMPRISING AN EXTRACT OF MOZUKU**

VERWENDUNG EINER ANTIOXIDATIVEN ZUSAMMENSETZUNG ENTHALTEND EINEN EXTRAKT AUS MOZUKU-ALGEN

UTILISATION D'UNE COMPOSITION ANTIOXYDANTE COMPRENANT UN EXTRAIT DE MOZUKU

(84) Designated Contracting States:
**AT BE CH CY DE DK ES FI FR GB GR IE IT LI LU MC NL PT SE TR**

(30) Priority: **26.05.2000 JP 2000155924**

(43) Date of publication of application:
**26.02.2003 Bulletin 2003/09**

(73) Proprietors:
• **KABUSHIKI KAISHA YAKULT HONSHA**
**Minato-ku,**
**Tokyo 105-8660 (JP)**
• **Tropical Technology Center Ltd.**
**Gushikawa-shi,**
**Okinawa 904-2234 (JP)**

(72) Inventors:
• **SONE, Toshiro**
**Minato-ku**
**Tokyo 105-8660 (JP)**
• **CHIBA, Katsuyoshi**
**Minato-ku**
**Tokyo 105-8660 (JP)**
• **IIZUKA, Ryoko**
**Minato-ku**
**Tokyo 105-8660 (JP)**
• **HANAMIZU, Tomoko**
**Minato-ku**
**Tokyo 105-8660 (JP)**

• **KONO, Hirosi**
**Minato-ku**
**Tokyo 105-8660 (JP)**
• **IHA, Masahiko**
**Gushikawa-shi**
**Okinawa 904-2234 (JP)**

(74) Representative: **Hartz, Nikolai**
**Wächtershäuser & Hartz**
**Patentanwälte**
**Weinstrasse 8**
**80333 München (DE)**

(56) References cited:
**EP-A- 0 908 467**    **EP-A- 1 234 568**
**JP-A- 10 053 514**    **JP-A- 11 021 247**
**JP-A- 11 228 602**

• **DATABASE CA [Online] CHEMICAL ABSTRACTS SERVICE, COLUMBUS, OHIO, US; OHWA, KYOSUKE: "Manufacture of mozuku (Nemacystus decipiens) extract"** retrieved from STN Database accession no. 130:80717 XP002241262 & JP 11 001437 A (HORIUCHI K. K., JAPAN) 6 January 1999 (1999-01-06)
• **PATENT ABSTRACTS OF JAPAN vol. 1998, no. 14, 31 December 1998 (1998-12-31) -& JP 10 245334 A (TAKO MASAKUNI), 14 September 1998 (1998-09-14)**

- PATENT ABSTRACTS OF JAPAN vol. 1999, no. 03, 31 March 1999 (1999-03-31) -& JP 10 330219 A (NOEVIR CO LTD), 15 December 1998 (1998-12-15)
- DATABASE WPI Derwent Publications Ltd., London, GB; AN 1985-180065 XP002241263 "Toilet soap containing seaweed extract" & JP 60 106899 A (DAIICHI SEIMO KK), 12 June 1985 (1985-06-12)
- CHANGHU XUE ET AL.: 'Antioxidative activities of several marine polysaccharides evaluated in a phosphatidycholine-liposomal suspension and organic solvents' BIOSCI. BIOTECHNOL. BIOCHEM. vol. 62, no. 2, 1998, pages 206 - 209, XP002944790
- DATABASE [Online] YOSHIKAWA TOSHIKAZU: 'Antioxidants in marine algae, kosanka busshitu no subete', XP002944791 & SENTAN IGAKUSHA 1998, TOKYO, pages 157 - 164
- MATSUKAWA R. ET AL.: 'A coparison of screening methods for antioxidant activity in seaweeds' J. APPL. PHYCOL. vol. 9, no. 1, 1997, pages 29 - 35, XP002944792

**Description**

Field of the Invention

The present invention describes an antioxidant containing *Mozuku* extract as an active ingredient that exhibits an excellent antioxidizing and anti-inflammatory effect in response to ultraviolet rays or a stimulator and relates, in particular, to the use of an antioxidant comprising an extract of Mozuku as an active component for the preparation of a composition for the prevention or treatment of inflammation.

Description of Background Art

Active oxygen is produced when the skin is exposed to ultraviolet rays. This is believed to be one of the causes of the inflammatory reaction that occurs thereafter. Conventionally, sunscreens have been used to prevent the inflammatory reaction because they exhibit an excellent effect. However, due to problems in sensation during use such as touch sensation and limitations to physical durability properties such as anti-chaffing and anti-sweat properties, these sunscreens have not been satisfactory in their capabilities of exhibiting a continuous preventive effect. A preparation that can prevent injury after exposure to ultraviolet rays has also been desired.

Steroidal anti-inflammatory agents have been used to reduce inflammation resulting from ultraviolet rays. Even though the curative effect of these agents has been confirmed, the steroids target many internal organs and result in serious side effects. As an alternative, various non-steroidal anti-inflammatory agents are used. However, these agents cannot easily be used because of a problem of causing stomach ulcers due to suppression of prostaglandin.

Also, there have been many reports regarding plant extracts. For example, glycyrrhiza extract, scutellaria root extract, chamomile extract (Japanese Patent Application Laid-open No. 1997/157172), spatholobus suberectus root extract (Japanese Patent Application Laid-open No. 1996/26965), astragalus root extract (Japanese Patent Application Laid-open No. 1996/73325), and the like have been used to reduce inflammation caused by ultraviolet rays. Theireffect, however, is not satisfactory.

On the other hand, there are many reports on skin preparations for external use and cosmetic compositions containing seaweed extract, especially *Mozuku* of *Phaeophyceae.* These preparations possess a whitening effect (Japanese Patent Application Laid-open No. 1991/251514), anti-wrinkle effect (Japanese Patent Application Laid-open No. 1997/268119), and moisturizing effect (Japanese Patent Publication No. 1996/766). Also, it is known that fucoidan, the active component of *Mozuku ,* exhibits an anti-allergic effect (Japanese Patent Application Laid-open No. 1999/21247) and a moisturizing effect (Japanese Patent Application Laid-open No. 1989/31707). These reports, however, mention neither the antioxidation effect nor the effect of preventing or curing inflammation resulting from ultraviolet rays.

From the viewpoint of side effects, pharmaceutical products presently used for the treatment of inflammation resulting from the production of active oxygen when the skin is exposed to ultraviolet rays cannot always be confidently used. Also, there have been very few plant extracts that could be used satisfactorily from the viewpoint of effectiveness and cost. Therefore, a low priced highly effective product that can be used on a day-to-day basis has been desired.

Accordingly, the object of the present invention is to provide an antioxidant effective in preventing and curing inflammation resulting from ultraviolet rays, for example, as a material that can be safely used on a day-to-day basis, and to provide a skin preparation for external use comprising this antioxidant.

DISCLOSURE OF THE INVENTION

In order to achieve the above object, the present inventors examined the antioxidation activities of various compounds and have discovered that the extract of *Mozuku* possesses an excellent antioxidation and anti-inflammation effect against ultraviolet rays or stimulators. The inventors have also discovered that the extract of *Mozuku* possesses an excellent sensation during use, thereby completing the present invention.

Specifically, the present invention provides a use of an antioxidant comprising an extract of *Mozuku* as an active component for the preparation of a composition for the treatment or prevention of inflammation.

The present invention also describes a skin preparation for external use comprising the above antioxidant.

BRIEF DESCRIPTION OF THE DRAWINGS

Figure 1 shows the test results of the radical scavenging effect of various kinds of seaweed extracts at various concentrations.
Figure 2 shows the test results of the SOD-like activity of *Mozuku* extract at various concentrations.

Figure 3 shows the test results of the TNFα secretion suppressing activity of *Nemacystis* extract after ultraviolet irradiation.

Figure 4 shows the test results of the mouse ear edema suppressing activity of *Mozuku* extract.

## DETAILED DESCRIPTION OF THE INVENTION AND PREFERRED EMBODIMENT

The extract obtained from *Mozuku* (hereinafter referred to as " *Mozuku* extract") used as the active component in the antioxidant of the present invention is extracted from the seaweed *Mozuku* using a suitable solvent such as water or alcohol.

As *Mozuku Tinocladia crassa* Kylyn, *Papenfussiella kuromo* Inagaki , *Cladosiphon okamuranus* Tokida, *Analipus japonicus* Wynnu, *Nemacystus decipiens* Kuckuck, *Heterosaundersella hattoriana* Tokida, *Sphaerotrichia divaricata* Kylin, *Myriogloea simplex* Inagaki can be used with cultivated *Cladosiphon okamuranus* Tokida being preferable from the effect and economic viewpoints. Dried, salt-cured, or frozen *Cladosiphon okamuranus* Tokida may be used.

The extract of *Mozuku* can be obtained by adding a suitable amount of solvent to the above *Mozuku* raw material at room temperature or under heating and allowing the raw material to soak. Examples of the solvent used include, in addition to water, ethanol, methanol, polyhydric alcohols such as propylene glycol, 1, 3-butylene glycol, and glycerol, as well as mixtures of these alcohols.

As the method for manufacturing the *Mozuku* extract, there are many methods that have been reported (Japanese Patent Publication No. 1996/766, Japanese Patent Application Laid-open Nos. 1998/245334 and 1999/1437, and the like). Any one of these methods may be used in the present invention. The following method can be given as a specific example for manufacturing *Mozuku* extract preferably used in the present invention. A solvent such as water or an acidic aqueous solution (pH 2.5-3.0) is added to the whole seaweed of frozen *Cladosiphon okamuranus Tokida.* The mixture is repeatedly extracted with heating, filtered, and desalted to obtain. *Mozuku* extract. The obtained extract is light yellow to light brown and has a slight peculiar odor. The obtained *Mozuku* extract is concentrated under reduced pressure at a temperature of 50°C or less. The concentrate may be frozen or spray-dried to obtain extract powder. If necessary, the extract may be fractionated using an appropriate separation means such as gel filtration, electrodialysis, silica gel column chromatography, HPLC, or the like to obtain fractions with high activity.

The *Mozuku* extract obtained in this manner may be independently used as an antioxidant for preventing or curing inflammation due to ultraviolet radiation. More preferably, however, the *Mozuku* extract is used as an antioxidant in combination with a suitable known pharmaceutical carrier.

The antioxidant containing the *Mozuku* extract obtained in this manner is a material derived from an edible seaweed and, therefore, is completely free of anything harmful. The antioxidant can be combined with a suitable known base for external preparation and used as a skin preparation for external use such as a cosmetic, drug, quasi-drug, or the like. The amount of antioxidant (the amount of the solid *Mozuku* extract) is 0.0001-30 wt% (hereinafter indicated as "%"), and particularly preferably 0.001-10% of the total amount of the preparation for external use.

The skin preparation for external use can be prepared by formulating, as required, additives commonly used in skin preparations for external use, such as other pharmaceutical components, water, alcohols, humectants, oil components, amphipathic compounds, thickeners, UV absorbers, chelating agents, pH controllers, antiseptic agents, coloring matters, perfumes, and the like, in addition to the antioxidant containing the *Mozuku* extract. The skin preparation for external use of the present invention may contain the above essential components in a lamellar structure or the preparation may be in the form of emulsion. Analgesics, antiphlogistics, bactericides, antiseptics, and the like can be given as other pharmaceutical components used in the preparation of the skin preparation for external use. Specific examples of analgesics and antiphlogistics include methyl salicylate, camphor, tannic acid, mefenamic acid, ibuprofen, benzydamine, hydrocortisone, and the like. As specific examples of bactericides and antiseptics, salicylic acid, ichthammol, chlorohexidine, gentamicin, and the like can be given. As humectants, hyaluronic acid, lactobacillus culture broth, polyhydric alcohols such as glycerol, propylene glycol, 1,3-butylene glycol, sorbitol, polyglycerol, polyethylene glycol, and dipropylene glycol, NMF components such as amino acids, sodium lactate, and sodium pyrrolidone carboxylate, water-soluble high molecular weight compounds such as collagen, mucopolysaccharides, and chondroitin sulfate, and the like can be given.

As oil components among the optional components, the following examples can be given: vegetable oils and fats such as castor oil, olive oil, cacao oil, camellia oil, coconut oil, Japan wax, jojoba oil, grape seed oil, and avocado oil; animal oils and fats such as mink oil and egg yolk oil; waxes such as beeswax, whale wax, lanolin, carnauba wax, and candelila wax; hydrocarbons such as liquid paraffin, squalane, micro-crystallin wax, ceresin wax, paraffin wax, and petroleum jelly; natural and synthetic fatty acids such as lauric acid, myristic acid, stearic acid, oleic acid, iso-stearic acid, and behenic acid; natural and synthetic higher alcohols such as cetyl alcohol, stearyl alcohol, hexyl decanol, octyl dodecanol, and lauryl alcohol; and esters such as isopropyl myristate, isopropyl palmitate, octyldodecyl myristate, octyldodecyl oleate, and cholesterol oleate.

The following compounds can be given as examples of amphipathic compounds: nonionic surfactants such as oleophillic glycerol monostearate, self-emulsifiable glycerol monostearate, polyglycerolmonostearate, sorbitanmonooleate, polyethylene glycol monostearate, polyoxyethylene sorbitan monooleate, polyoxyethylene cetyl ether, polyoxyethylene sterol, polyoxyethylene lanolin, polyoxyethylene bees wax, and polyoxyethylene hydrogenated castor oil; anionic surfactants such as sodium stearate, potassium palmitate, sodium cetyl sulfate, sodium lauryl phosphate, triethanolamine palmitate, polyoxyethylene sodium lauryl phosphate, and sodium N-acyl glutamate; cationic surfactants such as stearyl dimethylbenzyl ammonium chloride and stearyl trimethyl ammonium chloride; amphoteric surfactants such as alkylaminoethyl glycine chloride and lecithin; and the like.

As thickners, sodium alginate, xanthan gum, aluminum silicate, quince extract, naturally occurring polymers such as traganth gum and starch, half-synthetic polymers such as methylcellulose, hydroxyethylcellulose, carboxymethylcellulose, soluble starch, and cationized cellulose, synthetic polymers such as carboxy vinyl polymer and polyvinyl alcohol, and the like can be given. As UV absorbers or dispersion agents, 2-hydroxy-4-methoxybenzophenone, octyldimethyl p-aminobenzoate, ethylhexyl p-methoxycynamate, titanium oxide, kaolin, talc, and the like can be given. As vitamins, Vitamin A, vitamin B, vitamin C, vitamin D, vitamin E, vitamin F, vitamin K, vitamin P, vitamin U, carnitine, ferulic acid, γ-oryzanol, α-lipoic acid, orotic acid, derivatives of these vitamins, and the like can be given. As amino acids, glycine, alanine, valine, leucine, isoleucine, serine, threonine, phenylalanine, thyrosin, tryptophan, cystine, cysteine, methionine, proline, hydroxyproline, aspartic acid, glutamic acid, arginine, histidine, lysine, derivatives of these vitamins, and the like can be given.

As antiseptic agents, benzoate, salicylate, sorbate, dehydroacetate, p-oxybenzoate, 2,4,4'-trichloro-2'-hydroxydiphenyl ether, 3,4,4'-trichlorocarboanilide, benzalkonium chloride, hinokitiol, resorcin, ethanol, and the like can be given.

The skin preparation for external use of the present invention, comprising cosmetics, drugs, quasi-drugs, and the like as mentioned above, may be prepared in various forms such as a face lotion, milky lotion, moisturizing cream, cleansing cream, massage cream, face cream, pack, beauty solution, shampoo, body shampoo, body lotion, solid soap, and the like by conventional methods.

EXAMPLES

The present invention will be described in more detail by way of examples, reference examples, and test examples which should not be construed as limiting the present invention.

Reference Example 1

Preparation of seaweed extracts

(1) Preparation of *Mozuku* extract

2 1 of water was added to 1 kg of frozen stored *Cladosiphon okamuranus* Tokida and the mixture was adjusted to pH 3.0 by the addition of 2N HCl aqueous solution. The mixture was heated at 90°C for one hour while stirring to extract the seaweed essence. Immediately after extraction, the extract was neutralized to pH 5.0 with 2N sodium hydroxide and cooled. After removing impurities by filtration, the extract was desalted by electrodialysis and concentrated under reduced pressure to a Brix of 3% or more. The concentrated extract was freeze-dried to obtain about 20 g of *Mozuku* extract.

(2) Preparation of *Laminariales* extract

2 1 of water was added to 1 kg of frozen stored *Laminaria japonica* and the mixture was adjusted to pH 3.0 by the addition of 2N HCl aqueous solution. The mixture was heated at 90°C for one hour while stirring to extract the *Laminariales* essence. Immediately after extraction, the extract was neutralized to pH 5.0 with 2N sodium hydroxide and cooled. After removing impurities by filtration, the extract was desalted by electrodialysis and concentrated under reduced pressure to a Brix of 3% or more. The concentrated extract was freeze-dried to obtain about 20 g of *Laminariales* extract.

(3) Preparation of *Fucales* extract

2 1 of water was added to 1 kg of frozen stored *Pelvetia wrightii* and the mixture was adjusted to pH 3.0 by the addition of 2N HCl aqueous solution. The mixture was heated at 90°C for one hour while stirring to extract the *Fucales* essence. Immediately after extraction, the extract was neutralized to pH 5.0 with 2N sodium hydroxide and cooled. After removing impurities by filtration, the extract was desalted by electrodialysis and concentrated under reduced pressure to a Brix of 3% or more. The obtained concentrated extract was freeze-dried to obtain about 20 g of *Fucales* extract.

Test Example 1

Radical scavenging effect

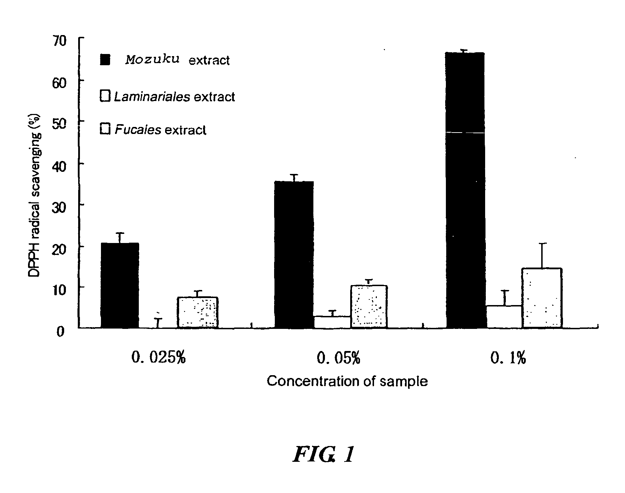

The radical scavenging effect of various seaweed extracts was examined as follows. The *Mozuku* extract, *Laminariales* extract, and *Fucales* extract were used as test specimens. 100 µl of a 20% ethanol solution of the test specimen, 50 µl of 200 mM acetic acid buffer solution (pH 5.5), and 100 µl of 250 µM 2,2-diphenyl-1-picrylhydrazyl picrylhydrazyl (DPPH; ethanol solution) were charged into wells and incubated at 30°C for 30 minutes to measure the absorbance at a wavelength of $OD_{515}$ nm. The radical scavenging rate (%) of DPPH was calculated from the following formula. A sample with a solvent added instead of the test specimen was used as a control. The results are shown in Figure 1.

$$\texttt{DPPH radical scavenging rate (\%) = \{(A-B)/A\}x100}$$

A: The $OD_{515}$ value of the control
B: The $OD_{515}$ value of the test specimen
Figure 1 clearly shows the DPPH radical scavenging effect of *Mozuku* extract. In addition, the *Nemacystis* extract was shown to exhibit a better antioxidizing effect than *Laminariales* extract and *Fucales* extract.

Test Example 2

Superoxide anion diminishing activity (SOD-like activity)

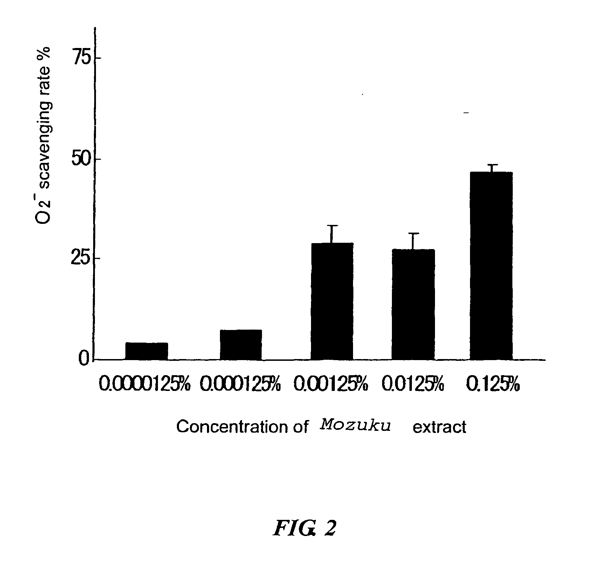

The SOD-like activity of *Nemacystis* extract was measured according to the SOD-test Wako (Wako Pure Chemical Industries, Ltd.) based on the principle of the nitro blue tetrazolium (NTB) reduction method using a 96-well microplate. Specifically, 10 µl of a solution of *Mozuku* extract with a prescribed concentration in 20% ethanol was added as a test specimen to 95 µl of a coloring reagent containing 0.24 mM NBT and 0.4 mM xanthine. After further addition of 95 µl of an enzyme (xanthine oxidase) solution, the mixture was incubated for 30 minutes at 37°C. The enzyme reaction was terminated with the addition of 100 µl of a reaction termination solution (69 mM sodium dodecyl sulfate) to measure the absorbance at a wavelength of $OD_{562}$ nm using a micro-plate reader. A sample prepared by adding purified water instead of the enzyme (a blank) and a solvent instead of the test specimen was used as a control. The superoxide anion ($O_2^-$) scavenging rate was calculated according to the following formula. The results are shown in Figure 2.

$$\texttt{O}_2^-\ \texttt{scavenging rate (\%) = \{(A-B)/A\}x100}$$

A: The $OD_{562}$ value of the control
B: The $OD_{562}$ value of the test specimen
The results of Figure 2 have made it clear that the $O_2^-$ scavenging rate of *Mozuku* extract is weak even at a low concentration, demonstrating superior scavenging activity of the *Nemacystis* extract.

Test Example 3

TNFα secretion controlling activity on human keratinocyte irradiated with UV light:

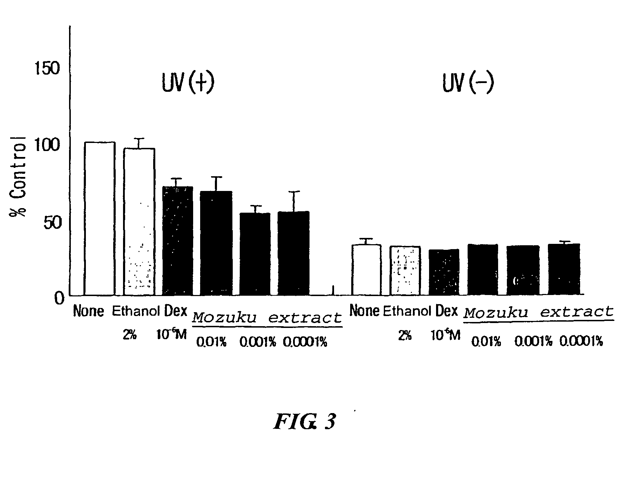

TNFα secretion controlling activity of the *Mozuku* extract effect on human keratinocyte irradiated with UV light was examined using normal human keratinocytes transformed with SV40 virus T-antigen as cells. The cells were cultured in a 5% $CO_2$ incubator at 37°C using HuMedia-KG2 (Kurabo Industries, Ltd.) as a culture medium. For differentiation induction, the cells were cultured for 24 hours by replacing the medium with a high concentration calcium culture medium (1 mM $CaCl_2$ added). The cells were irradiated with UVB for 25 seconds in PBS at a dose of 200 J/m$^2$ using a 312 nm UV lamp (VILBER LOURMAT Co.). Then, the cells were cultured in a usual culture medium or with the addition of a sample. The amount of TNFα in the culture medium supernatant liquid at 24 hours after irradiation was measured using a high sensitive ELISA kit (BIO SOURCE). The results are shown in Figure 3.

Figure 3 shows that the *Mozuku* extract controlled secretion of TNF$\alpha$ due to irradiation of UV rays at a concentration of 0.01-0.0001%. The effect was equivalent to or more than the effect obtained by dexamethasone (Dex), a $10^{-6}$ M anti-inflammatory steroid, used as a positive control. No toxicity to the cells was observed. In this manner, the *Mozuku* extract was found to control excessive secretion of TNF$\alpha$, which is known as an inflammatory cytokine, due to irradiation with UVB.

Test Example 4

Mouse ear swelling suppressing activity

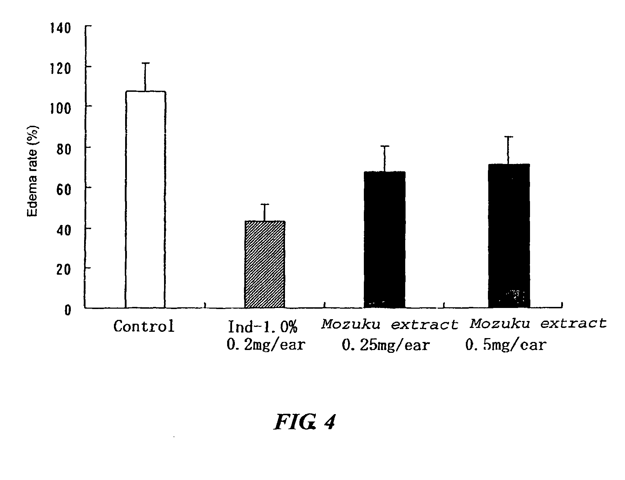

Mouse ear swelling suppressing activity of the *Mozuku* extract was examined as follows. The thickness of the ear of an ICR male mouse (age: 5 weeks) was measured using a thickness gauge (Digimatic Indicator 543, manufactured by Mitsutoyo Co., Ltd.). The *Mozuku* extract was applied to the ear in the amount of 0.25 mg/ear or 0.5 mg/ear. As a comparative test, an indomethacin solution, prepared by dissolving indomethacin at a concentration of 1.0% in 70% acetone, was applied to the mouse ear in the amount of 0.2 mg/ear. 30 minutes after the application, 10 $\mu$l of a 40 $\mu$l/ml solution of 12-O-tetradecanoylphorbol-13-acetate (TPA) in 70% acetone was applied to the in and out sides of the mouse ear to induce a skin reaction. The thickness of the ear skin was measured after 4 hours at which the value of the swelling became the maximum. The ratio of the measured thickness to the thickness of the ear skin before the edema induction was calculated as the ratio of edema. The thickness of both the right and left ear skins was measured and the values were averaged to determine the ear skin thickness of the mouse. The results are shown in Figure 4.

It can be seen from Figure 4 that edema was induced by applying TPA to the ear skin of the mouse, increasing the ear skin thickness two times or more. A remarkable edema suppressing effect was exhibited when indomethacin or *Mozuku* extract is applied 30 minutes before the TPA application. The same results were seen when the specimen was applied simultaneously with TPA. In this manner, the *Mozuku* extract exhibits a superior anti-inflammatory action not only against UV rays but also against a chemical stimulant.

Example 1

A face lotion of the following composition was prepared. Formulation:

| (1) | Ethanol | 5.0 (wt%) |
|-----|---------|-----------|
| (2) | 1,3-Butylene glycol | 2.0 |
| (3) | Hyaluronic acid | 0.2 |
| (4) | Polyoxyethylene hydrogenated castor oil | 0.05 |
| (5) | Methyl p-oxybenzoate | 0.1 |
| (6) | Perfume | 0.1 |
| (7) | *Mozuku* extract (solid components) | 0.01 |
| (8) | Distilled water | Balance |
|     | Total | 100.00 |

Method of preparation:

Components (3) and (7) were dissolved in component (8). Then, components (1), (2), (4), (5), and (6) were added to the solution and the mixture was sufficiently stirred to obtain a face lotion.

Example 2

A milky lotion of the following composition was prepared. Formulation:

| (1) | Stearic acid | 2.0 (wt%) |
|-----|--------------|-----------|
| (2) | Liquid paraffin | 5.0 |
| (3) | Squalane | 2.0 |
| (4) | Sorbitan monostearate | 1.5 |
| (5) | Polyoxyethylene(20) sorbitan monostearate | 2.0 |
| (6) | Butyl p-oxybenzoate | 0.05 |

Table continued

| (7) | Glycerol | 2.0 |
| --- | --- | --- |
| (8) | Methyl p-oxybenzoate | 0.1 |
| (9) | Perfume | 0.15 |
| (10) | *Mozuku* extract (solid components) | 0.03 |
| (11) | Distilled water | Balance |
| | Total | 100.00 |

Method of preparation:

Components (7), (8), and (10) were added to component (11). Then, components (1)-(6) were added to the mixture and emulsified at 80°C. After the addition of component (9), the emulsion was allowed to cool to room temperature to obtain a milky lotion.

Example 3

A cream of the following composition was prepared. Formulation:

| (1) | Liquid paraffin | 23.0 (wt%) |
| --- | --- | --- |
| (2) | Petroleum jelly | 7.0 |
| (3) | Cetanol | 1.0 |
| (4) | Stearic acid | 2.0 |
| (5) | Beeswax | 2.0 |
| (6) | Sorbitan monostearate | 3.5 |
| (7) | Polyoxyethylene(20) sorbitan monostearate | 2.5 |
| (8) | Butyl p-oxybenzoate | 0.05 |
| (9) | Hyaluronic acid | 0.1 |
| (10) | 1,3-Butylene glycol | 1.0 |
| (11) | Methyl p-oxybenzoate | 0.1 |
| (12) | Perfume | 0.15 |
| (13) | *Lactobacillus* culture broth | 5.0 |
| (14) | *Mozuku* extract (solid components) | 0.05 |
| (15) | Distilled water | Balance |
| | Total | 100.00 |

Method of preparation:

Components (10), (11), (13), and (14) were added to component (15). Component (9) was added to and dissolved in the mixture to obtain a dispersion. Then, components (1)-(8) were added to the dispersion to emulsify the mixture at 80°C. After the addition of component (12), the emulsion was allowed to cool to room temperature to obtain a cream.

Example 4

A shampoo of the following composition was prepared. Formulation:

| (1) | Triethanolamine lauryl sulfate | 8.5 (wt%) |
| --- | --- | --- |
| (2) | Polyoxyethylene(3) triethanolamine lauryl sulfate | 8.5 |
| (3) | Coconut oil fat fatty acid diethanol amide | 3.0 |
| (4) | Ethylene glycol monostearate | 2.0 |
| (5) | Butyl p-oxybenzoate | 0.05 |
| (6) | Hyaluronic acid | 0.1 |
| (7) | 1,3-Butylene glycol | 1.0 |

Table continued

| (8) | Methyl p-oxybenzoate | 0.1 |
|---|---|---|
| (9) | Perfume | 0.15 |
| (10) | *Lactobacillus* culture broth | 3.0 |
| (11) | *Mozuku* extract (solid components) | 0.5 |
| (12) | Distilled water | Balance |
| | Total | 100.00 |

Method of preparation:

Components (6), (7), (8), (10), and (11) were added to component (12). Then, components (1)-(5) were added to the mixture to emulsify at 80°C. After the addition of component (9), the emulsion was allowed to cool to room temperature to obtain a shampoo.

INDUSTRIAL APPLICABILITY

Since the *Mozuku* extract has both the antioxidizing activity and the anti-inflammatory activity, inflammation can be cured or prevented by using the *Mozuku* extract. In addition, the *Mozuku* extract is highly safe and therefore can be continuously used for a long period of time.

The antioxidant containing the *Mozuku* extract as an active ingredient of the skin preparation for external use containing the antioxidant is effective for preventing and curing inflammation due to UV radiation and the like.

**Claims**

1.  Use of an antioxidant comprising an extract of Mozuku as an active component for the preparation of a composition for the treatment or prevention of inflammation.

2.  The use according to claim 1, wherein said inflammation results from ultraviolet rays or stimulators.

3.  The use according to claim 1 or 2, whereby the composition contains an amount of solid Mozuku extract of from 0.0001 to 30 wt.% of the total amount of the preparation.

4.  The use according to any one of claims 1 to 3, whereby the composition further contains a pharmaceutically acceptable carrier.

**Patentansprüche**

1.  Verwendung eines Antioxidans, das einen Mozuku-Extrakt als aktive Komponente aufweist, zur Herstellung einer Zusammensetzung zur Behandlung oder Vorbeugung einer Entzündung.

2.  Verwendung nach Anspruch 1, worin die Entzündung von ultravioletten Strahlen oder Stimulantien hervorgerufen ist.

3.  Verwendung nach Anspruch 1 oder 2, wobei die Zusammensetzung eine Menge an festem Mozuku-Extrakt von 0,0001 bis 30 Gew.-%, bezogen auf die Gesamtmenge des Präparats, enthält.

4.  Verwendung nach einem der Ansprüche 1 bis 3, wobei die Zusammensetzung ferner eine pharmazeutisch annehmbare Trägersubstanz enthält.

**Revendications**

1.  Utilisation d'un antioxydant comprenant un extrait de Mozuku en tant que composant actif, pour la préparation d'une composition pour le traitement ou la prévention d'une inflammation.

2. Utilisation selon la revendication 1, pour laquelle ladite inflammation résulte de rayons ultraviolets ou de stimulateurs.

3. Utilisation selon la revendication 1 ou 2, pour laquelle la composition contient une quantité d'extrait solide de Mozuku de 0, 0001 à 30% en poids de la quantité totale de la préparation.

4. Utilisation selon l'une quelconque des revendications 1 à 3, pour laquelle la composition contient en outre un support pharmaceutiquement acceptable.

*FIG. 1*

**O₂⁻ scavenging rate %** (y-axis, values 0, 25, 50, 75)

Concentrations (x-axis): 0.0000125%  0.000125%  0.00125%  0.0125%  0.125%

Concentration of *Mozuku* extract

*FIG. 2*

**FIG. 3**

*FIG. 4*